# EUROPEAN PATENT APPLICATION

(11) **EP 2 589 962 A1**
(43) Date of publication of application: **08.05.2013**
(21) Application number: 12168003.7
(22) Date of filing: 15.05.2012
(51) Int. Cl.: G01N 33/543

(54) **Highly sensitive method of assaying troponin i**

(30) Priority: 04.11.2011 KR 20110114524
(71) Applicant: Samsung Electronics Co., Ltd., Suwon-si, Gyeonggi-do, 443-742 (KR)
(72) Inventor: Lee, Joon Hyung, 446-720 Gyeonggi-Do (KR); Lee, Soo Suk, 443-470 Gyeonggi-do (KR); Choi, Youn Suk, 446-728 Gyeonggi-Do (KR); Park, Jin Young, 790-310 Gyeongsangbuk-do (KR); Seo, Hye Jung, 200-883 Gangwon-do (KR); Lee, Jung Nam, 405-241 Incheon (KR); Han, Kyung Yeon, 138-170 Seoul (KR)
(74) Representative: Grünecker, Kinkeldey, Stockmair & Schwanhäusser

(57) **Abstract**

A highly sensitive method of assaying an analyte present in a sample using protamine is provided, as well as an immunoassay reagent and biosensor that reduces nonspecific binding by electrostatic interaction of protamine with fibrinogen present in the sample.

## Description

### CROSS-REFERENCE TO RELATED APPLICATION

This application claims the benefit of Korean Patent Application No. 10-2011-0114524, filed on November 4, 2011, and all the benefits accruing therefrom under 35 U.S.C. § 119 which is hereby incorporated by reference as if fully set forth herein.

### BACKGROUND

### 1. Field

This disclosure relates to an immunoassay reagent, a biosensor, and a method of assaying an analyte, which may assay an analyte present in a sample in a highly sensitive manner using protamine.

### 2. Description of the Related Art

Biochemical binding assays are widely used to determine the presence and concentration of analytes contained in biological specimens. Such assays are based on the concept of binding partners. An analyte of interest binds to an analyte binding agent (such as, for example, an antibody to the analyte or a receptor for the analyte), and the analyte and the analyte binding agent are thus referred to as "binding partners." When one of the binding partners is bound to a solid phase, the assay is referred to as a "heterogeneous assay." Such heterogeneous assays include, for example, the sandwich method, the indirect method, and the competitive method, all terms readily recognized in the art.

The sensitivity of an assay typically refers to the smallest mass of an analyte that generates a statistically significant change in the signal generated by the assay when compared with the signal reading obtained in the absence of the analyte. Increased sensitivity is desirable because it permits detection of smaller amounts of analyte as well as an overall higher precision measurement of an analyte.

Non-specific binding refers to non-specific interactions of the binding partners in a heterogeneous assay system with a solid phase. Non-specific binding often reduces the sensitivity of heterogeneous assays, and it is therefore desirable to reduce such non-specific binding.

There remains a need for immunoassay reagents, biosensors, and assay methods, which allow the detection of an analyte in a highly sensitive manner.

### SUMMARY

Disclosed herein provides an immunoassay reagent for highly sensitive detection of an analyte, wherein the immunoassay reagent includes (a) a sample comprising the analyte and fibrinogen, and (b) protamine. Disclosed also provides a biosensor comprising (a) a substrate to which a capture antibody specific to an analyte is fixed, (b) an immunoassay reagent comprising (i) a sample comprising the analyte and fibrinogen, and (ii) protamine, and (c) a detection antibody specific to the analyte.

Disclosed further provides a highly sensitive method of assaying an analyte comprising: preparing an immunoassay reagent comprising (i) a sample comprising the analyte and fibrinogen and (ii) protamine, adding a detection antibody to the immunoassay reagent to provide a detection antibody-analyte complex, contacting the detection antibody-analyte complex with a capture antibody immobilized on a substrate to provide a detection antibody-analyte-capture antibody complex, and detetecting the analyte.

Disclosed further provides a method for detecting an analyte comprising: preparing an immunoassay reagent comprising (i) a sample comprising an analyte and fibrinogen, and (ii) a protamine; contacting the sample with a capture antibody immobilized on a substrate to provide a capture antibody-analyte complex; contacting the capture-antibody analyte complex with a detection antibody to provide a capture antibody-analyte-detection antibody complex; and detecting the analyte.

### BRIEF DESCRIPTION OF THE DRAWINGS

Exemplary embodiments are described in further detail below with reference to the accompanying drawings. It may be understood that various aspects of the drawings may have been exaggerated for clarity:

FIG. 1 is a schematic diagram of a gold staining assay; and

FIG. 2 is a schematic diagram of an enzyme-linked immunosorbent assay.

### DETAILED DESCRIPTION

Provided herein are immunoassay reagents comprising (i) a sample comprising analyte of interest and fibrinogen, and (b) a protamine, as well as methods of using the reagent for the detection of an analyte. Without wishing to be bound by any particular theory or mechanism of action, it is believed that the presence of fibrinogen in a sample, such as a blood sample, reduces the sensitivity and/or specificity of binding-partner based analyte detection methods. It is further believed that non-specific binding, or other modes of interference with such an assay, can be reduced by combining the sample with a protamine to provide an immunoassay reagent prior to detection. More specifically, it is believed that the improved performance is due to electrostatic interaction of protamine with fibrinogen present in the sample, thereby markedly improving the sensitivity and reproducibility of assays of the analyte.

According to one aspect of disclosed, the analyte is Troponin I, and disclosed provides a highly sensitive method of assaying Troponin I.

In the present specification, the term "Troponin I (TnI)" refers to a protein that is normally found in muscular tissue in conjunction with Troponin T and Troponin C. Troponin I regulates the calcium dependent interaction of actin and myosin. Three isotypes of Troponin I have been identified: slow-twitch skeletal muscle isoform Troponin I, fast-twitch skeletal muscle isoform Troponin I, and cardiac Troponin 1. When compared to the other Troponinins, cardiac Troponin I has 31 additional amino acid residues at the N-terminus and is the only Troponin isoform present in the myocardium.

Clinical studies have demonstrated that cardiac Troponin I (cTnI) is detectable in the bloodstream 4 to 6 hours after an acute myocardial infarction (AMI) and remains elevated for several days thereafter (see Mair et al., Clin. Chem., 41(9): 1266-1272 (1995); and Larue et al., Clin. Chem., 39(6): 972-979 (1993)). Thus, cTnI elevation covers the diagnostic windows of both creatine kinase-MB (CK-MB) and lactate dehydrogenase (see Bodor, J. Clin. Immunoassay, 17(1): 40-44 (1994)). Further studies have indicated that cTnI has a higher clinical specificity for myocardial injury than does CK-MB (see Adams et al., Circulation, 88(1): 101-106 (1993); and Apple et al., Clip. Chim. Acta 237: 59-66 (1995)).

Because of its cardiac specificity and sensitivity, cTnI has been used as a reliable marker in diagnosing patients with unstable angina and non-ST segment elevation acute coronary syndrome (ACS). Previous clinical studies of patients with ACS have shown that minor increases in cTnI values provide prognostic information about the short and long term risk of death (see Lindahl et al., J. Am. Coll, Cardiol., 38: 1497-1498, (2001); Venge et al., Am. J Cardiol., 89: 1035-1041 (2002); Galvani et al., Circulation, 95: 2053-2059 (1997); Antman et al., N. Eng. J. Med., 335: 1342-1349 (1996); Ottani et al., Am. Heart J, 40: 917-927 (2000); and Heidenreich et al., J. Am. Coll. Cardiol., 38: 478-485 (2001)). Ultimately, the assessment of the prognosis may be useful in identifying patients most likely to benefit from specific therapeutic interventions.

However, some factors, such as proteins, glucoses, and ions, that are contained in samples may hinder antigen-antibody binding during immunoassays of TnI. In particular, fibrinogen, which is a protein present in an amount of approximately 1.5 to 4.0 g/L in human blood plasma, greatly hinders antigen-antibody reactions, particularly TnI-antibody reactions. Thus, disclosed provides a method of reducing non-specific binding by removing fibrinogen contained in a sample by combining the sample with protamine, and thereby improving the sensitivity and reproducibility of assays of TnI using the sample.

According to an exemplary embodiment, disclosed provides an immunoassay reagent for a highly sensitive assay of TnI. The immunoassay reagent includes (a) a sample containing TnI and fibrinogen and (b) protamine. As mentioned, it is believed that a protamine-fibrinogen complex is produced due to electrostatic interaction of the protamine with fibrinogen present in the sample. Thus, the fibrinogen and protamine in the immunoassay can be present in the immunoassay reagent, in whole or in part, as a complex.

As used herein, the term "sample" refers to a wide range of biological substances and also compositions induced or extracted from the biological substances. The sample containing an analyte can be any suitable sample, including but not limited to a blood sample, such as a serum sample or plasma sample.

Blood plasma is the liquid component of blood in which the blood cells are suspended. For example, blood plasma can contain water, plasma proteins, lipids, saccharides, inorganic salts, and non-proteinous nitrogen compounds, such as urea, amino acids, and uric acids. Blood plasma can be separated from other components in a blood sample (e.g., blood cells) by any suitable means (e.g., using centrifugation). Plasma samples are obtained from blood tubes which contain anticoagulants such as sodium heparin, sodium citrate, sodium fluoride, and potassium oxalate or potassium ethylenediamine tetraacetic acid (EDTA). In one exemplary embodiment, a plasma sample is used.

As used herein, the term "analyte" refers to a material or chemical component to be measured. In one exemplary embodiment, the analyte is Troponin, such as Troponin I. In a preferred embodiment, the analyte is cardiac Troponin I.

As used herein, the term "fibrinogen" refers to a factor which is present in vertebrate plasma and lymph and plays a pivotal role in blood clotting. Fibrinogen is a glycoprotein having a molecular weight of approximately 340 kiloDaltons (KDa). In blood plasma, fibrinogen typically has a concentration of approximately 1.5 to 4.0 g/L. Fibrinogen is a hexamer containing two sets of three different chains (α(A), β(V), γ), which are linked to each other via disulfide bonds. The N-terminal sections of these three chains contain the cysteines that participate in the cross-linking of the chains. The C-terminal section contains a domain of approximately 225 amino acid residues, which may serve as molecular recognition units.

As used herein, the term "protamine" refers to a protein with multiple positive ions having an average charge amount of +20 and represented by Formula 1:

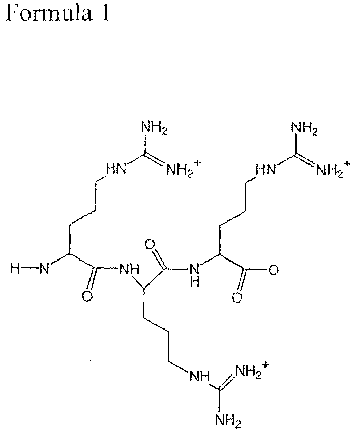

Protamine can be used in any suitable amount in the inventive immunoassay reagent, biosensor, and assay method. For example, protamine can be used in an amount of approximately 2 ng/ml to approximately 4 mg/ml, or an amount of approximately 1 mg/ml to approximately 3 mg/ml, or an amount of approximately 1.5 mg/ml to approximately 2.5 mg/ml. In one exemplary embodiment, 2.5 mg/ml of protamine is used.

According to another exemplary embodiment, a biosensor for use with the immunoassay reagent is provided. The biosensor comprises (a) a substrate to which a capture antibody (analyte-binding agent) specific to an analyte is fixed (i.e., affixed or immobilized), (b) an immunoassay reagent comprising (i) a sample comprising the analyte and fibrinogen and (ii) protamine, and (c) a detection antibody identical with the capture antibody fixed to the substrate. As previously discussed, the protamine is believed to form a complex with fibrinogen from the sample. Thus, the fibrinogen and protamine may be present in the immunoassay reagent, in whole or in part, as a fibrinogen-protamine complex.

As used herein, the term "biosensor" refers to an apparatus capable of qualitatively or quantitatively detecting an analyte using a specific recognition reaction of the analyte. Biosensors can be classified into a mass-based sensor, an optical sensor, an electrical sensor, and a magnetic sensor depending on the method of detecting an analyte. The mass-based sensor includes, but is not limited to, a quartz crystal microbalance (QCM), a cantilever sensor, and a surface acoustic wave (SAW) sensor. The optical sensor includes, but is not limited to, a sensor using ultraviolet (UV)-visible spectrometry, colorimetry, and surface plasmon resonance (SPR). The electrical sensor includes, but is not limited to, an electrochemical sensor and a field-effect-transistor (FET) sensor. The magnetic sensor includes, but is not limited to, a magnetic force microscopy (MFM) sensor. In one exemplary embodiment, a SAW sensor is used.

As used herein, the term "substrate" refers to a thin sheet formed of a material that is not magnetic and is unlikely to affect magnetic nanoparticles. The substrate can be at least one selected from the group consisting of a silicon wafer, a glass substrate, a quartz substrate, a metal substrate, and a plastic substrate, but the present invention is not limited thereto. In one exemplary embodiment, a silicon wafer is used.

As used herein, the term "analyte binding agent" refers to an antibody against the analyte or a receptor for the analyte, and the analyte and the analyte binding agent are referred to as "binding partners." The antibody can be a monoclonal or polycolonal. The antibody can be a chimeric antibody or hybrid antibody with dual or multiple antigen or epitope specificities. As used herein, the term "antibody" also encompasses single chain antibodies and antibody fragments, such as F(ab') 2, Fab', Fab, scFv, and the like. The antibody can be a commercially available antibody or can be prepared by techniques known in the art (See Harlow and Lane. Antibodies, A Laboratory Manual. Cold Spring Harbor Publications, New York (1988)).

In one embodiment, the antibody is an anti-Troponin I antibody, a polyclonal anti-Troponin I antibody, or a chemically or genetically engineered antibody thereof. For example, the monoclonal anti-Troponin I antibody is selected from the group including clones A34500 (binding to amino acids 87-91 of Troponin I), 81-87 (a34780, binding to amino acids 136-154 of Troponin I), A34650 (binding to amino acids 41-49 of Troponin I), 267 (ab 14530, binding to amino acids 169-184 of Troponin I), 16A11 (a24460, binding to amino acids 87-91 of Troponin I), 19C7 (a19615, binding to amino acids 41-49 of Troponin I), 560 (binding to amino acids 83-93 of cTnI) or combinations thereof, but is not limited thereto. In one exemplary embodiment, 16A11 is used as the anti-Troponin I antibody.

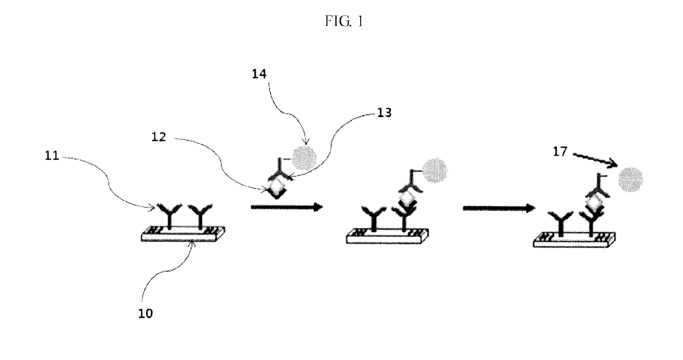

In one embodiment, the biosensor is used after the mass of an analyte is reinforced using a gold staining assay. A method of assaying Troponin I using a gold staining assay method is shown in FIG. 1.

FIG. 1 illustrates a capture antibody 11 fixed on a substrate 10. A detection antibody 13 bound to gold (Au) nanoparticles 14 is added to an immunoassay reagent from which fibrinogen present in a sample is removed or complexed by protamine to produce a "detection antibody (13)-Troponin I (12) complex." Next, the detection antibody (13)-Troponin I (12) complex is bound to the capture antibody 11 to produce a "detection antibody (13)-Troponin I (12)-capture antibody (11) complex," and Au⁺³ ions of HAuCl₄ and an NH₂OH reductant are added. The Au⁺³ ions are reduced to the gold nanoparticles by the NH₂OH reductant to produce mass-reinforced Au nanoparticles 17 so that a signal can be amplified. Thereafter, a variation in phase may be measured by a SAW sensor.

According to the above-described biosensor, analytes can be rapidly assayed with high sensitivity, and assay reproducibility can be remarkably improved. Thus, the analytes can be assayed efficiently even at low concentrations.

According to another exemplary embodiment, a highly sensitive method of assaying an analyte using the immunoassay reagent is provided. The method includes (a) preparing an immunoassay reagent comprising (i) a sample comprising the analyte and fibrinogen and (ii) protamine (b) adding a detection antibody to the immunoassay reagent to produce a detection antibody-analyte complex, (c) binding the detection antibody-analyte complex with a capture antibody immobilized on a substrate, thereby producing a detection antibody-analyte-capture antibody complex, and (e) detecting the analyte.

Alternatively, the sample can be captured on a substrate prior to labeling with a detection antibody. In this embodiment, the method comprises (a) preparing an immunoassay reagent comprising (i) a sample comprising the analyte and fibrinogen, and (ii) protamine; (b) contacting the immunoassay reagent with a capture antibody immobilized on a substrate to form a capture antibody-analyte complex, and (c) contacting the capture antibody-analyte complex with a detection antibody to form a capture antibody-analyte-detection antibody complex, and (d) detecting the analyte.

In some embodiments, the capture antibody is the same as (identical to) the detection antibody. In other embodiments, the capture antibody is different from the detection antibody, although both are prefably specific for the analyte.

The immunoassay reagent can be prepared by combining a sample containing an analyte of interest (e.g., TnI) and fibrinogen with protamine. The protamine is believed to complex with fibrinogen contained in the sample. Thus, the protamine and fibrinogen may be present in the immunoassay reagent, in whole or in part, as a protamine-fibrinogen complex. The assay may be performed with an immunoassay containing the complex, or the complex may be removed from the immunoassay reagent before proceeding with the assay. In other words, in the method of assaying an analyte according to disclosed, the step of preparing an immunoassay reagent can further comprise removing some or all protamine-fibrinogen complexes from the immunoassay reagent before proceeding with steps (b), (c), and (d) as set forth herein.As used herein, the term "assay method" refers to a method of verifying the presence, absence, or amount of an analyte. Detecting the analyte can comprise detecting the presence of the capture antibody-analyte-detection antibody complex, wherein the presences of such a complex indicates the presence of the analyte. Such detection can be performed by any suitable technique.

The assay method includes, but is not limited to, an enzyme-linked immunosorbent assay (ELISA) method, an electro-chemiluminescent assay (ECL) method, a radioimmunoassay (RIA) method, a solid-phase RIA (SPRIA) method, an immunoblotting method, an immunoprecipitation method, an immunohistochemical staining method, or a fluorescent activated cell sorting (FACS) method. In one exemplary embodiment, an ELISA method is used.

In a sandwich immunoassay, an analyte is captured by a capture antibody fixed onto a substrate. The fixing of the capture antibody can be accomplished using a known technique, for example, by use of a streptavidin-coated substrate. The capture antibody can be directly or indirectly labeled. For example, the capture antibody can be labeled with biotin and bound to a substrate coated with streptavidin. Alternatively, the capture antibody can be unlabeled and a target analyte can be bound to a labeled detection antibody after being bound to the capture antibody.

Examples of labels for use in disclosed include, but are not limited to, known labels, such as luciferase, luciferin, maleate dehydrogenase, urease, peroxidase such as horseradish peroxidase (HRP), alkaline phosphatase (AP), β-galactosidases, glucoamylases, lysozymes, saccharide oxidases, hetorocyclic oxidases, lactoperoxidases, and microperoxidases, and a combination thereof. The HRP oxidizes orthophenylene diamine (OPD) or 3,3',5,5'-tetramethylbenzidine hydrochloride (TMB), and the AP oxidizes para-nitrophenyl phosphate. In one exemplary embodiment, an HRP label and TMB are used.

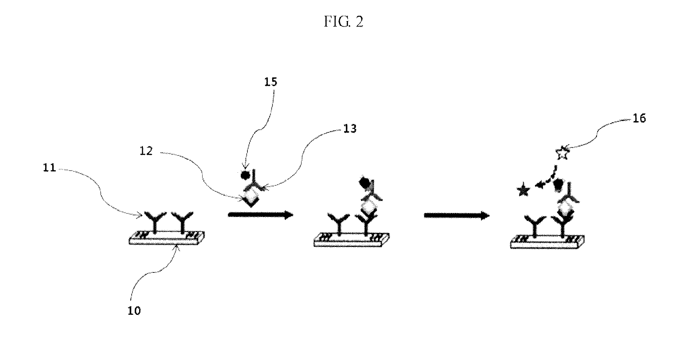

The assay method can be performed using an ELISA method. A method of detecting Troponin I using an ELISA method is shown in FIG. 2.

To begin with, a capture antibody 11 is fixed onto a substrate 10. A detection antibody 13 labeled with HRP enzyme 15 is added to an immunoassay reagent from which fibrinogen present in a sample is removed by protamine to produce a "detection antibody (13)-Troponin I (12) complex." Next, the detection antibody (13)-Troponin I (12) complex is bound to a capture antibody 11 to produce a "detection antibody (13)-Troponin I (12)-capture antibody (11) complex." After TMB 16 is added, the intensity of light is measured by using a chromogenic reaction.

According to the assay method, an analyte can be rapidly detected with high sensitivity. For example, according to the above-described assay method, the sensitivity of the assay is greater using an immunoassay reagent comprising protamine as compared to the same assay without protamine, that is, the assay sensitivity of an analyte, such as Troponin I, is high and remarkably improved, for example, at least two, three, four, or five times higher than when the inventive immunoassay reagent is not used. Furthermore, according to the above-described assay method, the reproducibility of assays of an analyte, such as Troponin I, is markedly improved, for example, at least two, three, or four times higher than when the inventive immunoassay reagent is not used.

Hereinafter, various embodiments are provided only for descriptions, and the scope of protection is not limited to the embodiments set forth herein.

### EXAMPLE 1

This example demonstrates the effects of protamine in an immune reaction using ELISA.

25 mg/ml of protamine is added to plasma separated from the human body, and centrifuged to extract a supernatant. ELISA is performed on the extracted supernatant. In this case, 16A11 labeled with HRP is used as a detection antibody, and the intensity of a luminous signal due to a TMB oxidation reaction is identified. The results are shown in Table 1.

**Table 1.**

| **Protamine (mg/ml)** | **Troponin I (0 ng/ml)** | **Troponin I (100 ng/ml)** | **Slope** |
|---|---|---|---|
| 0 | 0 00625 | 0.0115 | 0.0000525 |
| CV(%) | 35.4 | 24.7 | |
| 2.5 | 0 0084 | 0 09405 | 0 0008565 |
| CV (%) | 10.8 | 11 3 | |

| | | | |
|---|---|---|---|
| CV: coefficient of variation | | | |

Referring to Table 1, when protamine is added, all luminous signals increase more than when protamine is not added. Also, CV% is reduced from 35.4 and 24.7 to 10.8 and 11.3, respectively, demonstrating that assay reproducibility is markedly improved.

### EXAMPLE 2

This example demonstrates the effects of protamine in an immune reaction using a gold staining assay.

25 mg/ml of protamine is added to plasma separated from the human body, and centrifuged to extract a supernatant. A gold staining assay is performed on the extracted supernatant. In this case, Au ions are reduced to Au nanoparticles bound to a detection antibody so that the size of the Au nanoparticles increase to obtain mass-reinforced Au nanoparticles. Thus, a slope of variation in phase is measured using a SAW sensor. The results are shown in Table 2.

**Table 2.**

| **Protamine (mg/ml)** | **Troponin I (0 ng/ml)** | **Troponin I (10 ng/ml)** | **Slope** |
|---|---|---|---|
| 0 | 3 | 6.2 | 0.32 |
| CV (%) | 25.9 | 23.1 | |
| **2.5** | 12.4 | 57 | 4.46 |
| CV (%) | 8.3 | 7.9 | |

Referring to Table 2, when protamine is added, all phase slopes increase more than when protamine is not added. Also, CV% is reduced from 25.9 and 23.1 to 8.3 and 7.9, respectively, demonstrating that assay reproducibility is markedly improved.

As is evident from the results of Examples 1 and 2, when protamine is added, the sensitivity and reproducibility of assays of an analyte, such as Troponin 1, are markedly improved.

While exemplary embodiments have been disclosed herein, it should be understood that other variations may be possible. Such variations are not to be regarded as a departure from the scope of exemplary embodiments, and all such modifications as would be obvious to one skilled in the art are intended to be included within the scope of the following claims.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An immunoassay reagent for detection of an analyte, comprising (a) a sample containing the analyte and fibrinogen and (b) protamine, wherein the fibrinogen and protamine can be part of a protamine-fibrinogen complex.

2. The reagent according to claim 1, wherein the analyte is Troponin 1.

3. The reagent according to claim 1 or 2, wherein the sample is blood plasma.

4. The reagent according to any one of claim 1s to 3, wherein the protamine is contained in an amount of approximately 2 nanogram/milliliter to approximately 4 milligram/milliliter.

5. A biosensor comprising:
(a) a substrate to which a capture antibody specific to an analyte is fixed;
(b) an immunoassay reagent according to any one of claims 1 to 4; and
(c) a detection antibody identical with the capture antibody fixed to the substrate.

6. The biosensor according to claim 5, which is a mass-based sensor, an optical sensor, an electrical sensor, or a magnetic sensor.

7. The biosensor according to claim 5 or 6, wherein the substrate is at least one selected from the group consisting of a silicon wafer, a glass substrate, a quartz substrate, a metal substrate, and a plastic substrate.

8. A method of detecting an analyte, comprising:
preparing an immunoassay reagent according to any one of claims 1 to 4;
adding a detection antibody to the immunoassay reagent to produce a detection antibody-analyte complex;
binding the detection antibody-analyte complex with a capture antibody immobilized on a substrate, thereby producing a detection antibody-analyte-capture antibody complex; and
detecting the analyte.

9. The method according to claim 8, wherein detecting the analyte comprises the use of enzyme-linked immunosorbent assay (ELISA), electro-chemiluminescent assay (ECL), radioimmunoassay (RIA), a solid-phase RIA (SPRIA), immunoblotting, immunoprecipitation, immunohistochemical staining, or a fluorescent activated cell sorting (FACS).

10. The method according to claim 8 or 9, wherein the capture antibody or detection antibody is labeled with luciferase, luciferin, maleate dehydrogenase, urease, peroxidase, alkaline phosphatase (AP), β-galactosidase, glucoamylase, lysozyme, saccharide oxidase, hetorocyclic oxidase, lactoperoxidase, or microperoxidase.

11. The method of any one of claims 8 to 10, further comprising fixing a capture antibody specific to the analyte to a substrate.

12. The method of any one of claims 8 to 11, wherein the capture antibody and the detection antibody are the same.

13. The method of any one of claims 8 to 12, wherein preparing the immunoassay reagent comprises combining a sample comprising an analyte and fibrinogen with protamine to form protamine-fibrinogen complexes.

14. The method of any one of claims 8 to 13, wherein the method further comprises removing the protamine-fibrinogen complexes from the immunoassay reagent prior to contacting the immunoassay reagent with a detection antibody or a capture antibody.

15. A method of detecting an analyte, comprising:
preparing an immunoassay reagent according to any one of claims 1 to 4;
contacting the immunoassay reagent with a capture antibody immobilized on a substrate to form a capture antibody-analyte complex;
contacting the capture antibody-analyte complex with a detection antibody to form a capture antibody-analyte-detection antibody complex; and
detecting the analyte.
